# EUROPÄISCHE PATENTSCHRIFT

(11) **EP 3 249 400 B1**
(45) Veröffentlichungstag und Bekanntmachung des Hinweises auf die Patenterteilung: **25.12.2019**
(21) Anmeldenummer: 16171789.7
(22) Anmeldetag: 27.05.2016
(51) Int. Cl.: B01F 7/00, B01F 15/04, G05D 11/02

(54) **VORRICHTUNG UND VERFAHREN ZUR ISOLATION VON PARASITEN AUS ORGANISCHEM GEWEBE**
DEVICE AND METHOD FOR THE ISOLATION OF PARASITES FROM ORGANIC TISSUE
DISPOSITIF ET PROCEDE D'ISOLATION DES PARASITES PROVENANT D'UN TISSU ORGANIQUE

(43) Veröffentlichungstag der Anmeldung: 29.11.2017
(73) Patentinhaber: Moritz, Thomas, 25462 Rellingen (DE)
(72) Erfinder: Moritz, Thomas, 25462 Rellingen (DE)
(74) Vertreter: Seemann & Partner Patentanwälte mbB

(56) Entgegenhaltungen:
- EP-A2- 0 123 316
- WO-A1-2006/034716
- CH-A- 273 342
- DE-C1- 4 004 537
- FR-A- 1 223 033
- H.R GAMBLE ET AL: "International Commission on Trichinellosis: Recommendations on methods for the control of Trichinella in domestic and wild animals intended for human consumption", VETERINARY PARASITOLOGY., Bd. 93, Nr. 3-4, 1. Dezember 2000 (2000-12-01), Seiten 393-408, XP055301075, NL ISSN: 0304-4017, DOI: 10.1016/S0304-4017(00)00354-X

## Beschreibung

Die Erfindung betrifft eine Vorrichtung zur Isolation von Parasiten aus organischem Gewebe. Die Erfindung betrifft ferner ein entsprechendes Verfahren zum Betreiben einer Vorrichtung zur Isolation von Parasiten aus organischem Gewebe.

Eine Vorrichtung und ein entsprechendes Verfahren zur Erzeugung bzw. Freisetzung und Abtrennung von Substanzen oder partikulären Gebilden aus flüssiger, plastischer oder fester Materie und die Verwendung einer derartigen Vorrichtung sind aus EP 0 123 316 B1 bekannt.

Ein alternatives Verfahren zum Nachweis von im Wesentlichen intakten kapselbildenden oder nicht-kapselbildenden Parasiten in Fleisch, das die Mazeration des Fleisches mit einer basischen Verdauungslösung vorsieht, ist aus EP 2 443 455 B1 bekannt.

Bei dem erstgenannten Verfahren gemäß EP 0 123 316 B1 werden Fleischproben aus Schweinen genommen, die Fleischproben in einem Gefäß zerkleinert und mit 0,5%iger Salzsäurelösung oder einer sauren Pufferlösung zusammen mit dem Enzym Pepsin zugeführt. Die Lösung wird dann erwärmt, so dass ein künstlicher Verdauungsprozess vorgesehen ist. Das verdaute Fleisch wird dann durch ein Sieb bzw. einen Filter hindurchgepresst. Die auf dem Filter zurückbleibenden Partikel können Parasiten, wie beispielsweise Trichinen, sein.

Die andere Lösung gemäß EP 2 443 455 B1 sieht eine künstliche Verdauung im basischen Milieu mit aktiven Proteasen vor. Hierbei ist das Verdauungsenzym eine Serin-Endopeptidase und die Verdauungslösung hat einen pH-Wert zwischen 7,3 und 10. Auch hier geht es darum, Trichinen in Schweinefleisch nachzuweisen. Das Verfahren gemäß EP 2 443 455 B1 sieht das mechanische Zerkleinern des zu untersuchenden Fleisches, die Mazeration des zu untersuchenden Fleisches durch Zugabe einer Verdauungslösung, die Mazeration unter gleichzeitiger Bewegung des Verdauungsansatzes und/oder gleichzeitiger Ultraschall-Behandlung, die Inaktivierung der Mazeration, die Filtration des Mazerats und die Kontrolle, Nachweis, Diagnostik und/oder Typisierung auf Befall mit Parasiten vor.

EP 2 443 455 B1 beschreibt die unterschiedlichen wesentlichen Methoden, um Trichinen bzw. Trichinella der verschiedenen Sorten in Tieren nachzuweisen. Eine der dort genannten Methoden ist eine mechanisch unterstützte Methode der künstlichen Verdauung, bei der das die Larven umgebende Muskelgewebe mittels Imitation der natürlichen Verdauung unter Zusatz von Pepsin im sauen Milieu künstlich verdaut, um die Larven dabei freizusetzen. Anschließend werden die Larven mikroskopisch untersucht. Die Trichinenschau, die auf diese Art durchgeführt wird, wird durch die Verordnung VO (EG) Nr. 2075/2005 der Kommission vom 05.12.2005 mit spezifischen Vorschriften für die amtlichen Fleischuntersuchungen auf Trichinen geregelt. Hierbei wird das Enzym Pepsin, eine Aspartat-Endopeptidase, verwendet, um die natürliche Verdauung zu imitieren. Als Alternative zu Pepsin kann auch Bromelin, Trypsin oder Papain verwendet werden. Diese Enzyme benötigen zur Verdauung ein saures bis neutrales Milieu. Das Enzym gemäß EP 2 443 455 B1 benötigt ein basisches Milieu zur Verdauung.

Eine weitere Vorrichtung zur Untersuchung von Schweinefleisch auf den Befall von Trichinen ist in DE 40 04 537 C1 offenbart.

WO 2006/034716 A1 beschreibt ein Verfahren zum Nachweis von Trichinen in Fleischproben.

In FR 1 223 033 A ist ein Messersatz für Schneidvorrichtungen offenbart, der Klingen unterschiedlicher Größe, Form und Ausrichtung umfasst.

CH 273 342 A zeigt einen Messerkopf für Vorrichtungen mit mechanischem Antrieb. Jedes Messer des Messerkopfs weist mindestens zwei sensenartig geformte Flügel mit in Richtung des Drehsinns zeigenden Spitzen auf. Die Schneiden der Messer verlaufen bis zum größten Durchmesser der Flügel.

Auch in dem Artikel "International Commission on Trichinellosis: Recommendations on methods for the control of Trichinella in domestic and wild animals intended for human consumption" der Zeitschrift "veterinary parasitology" werden Verfahren und Vorrichtungen zum Nachweis von Trichinen in verschiedenen Fleischproben beschrieben.

All die genannten Verfahren und Vorrichtungen haben das Problem, dass nur geringe Mengen an Fleisch untersucht werden können und zudem die Verfahrensdauer relativ lang ist.

Zudem war bei dem bisherigen Verfahren beschränkend, dass lediglich beispielsweise Fleisch von 35 Schweinen gleichzeitig in einer Vorrichtung auf Trichinen untersucht werden konnte bzw. Trichinen aus Fleischproben von 35 Schweinen entsprechend mit der Vorrichtung isoliert werden konnten.

Demgegenüber ist es Aufgabe der vorliegenden Erfindung, eine effiziente Untersuchung von organischem Gewebe, insbesondere Schweinefleisch oder Fisch auf Parasiten anzugeben, das eine größere Menge als bisher möglich erlaubt und zudem schnell und sicher durchgeführt werden kann.

Diese Aufgabe wird gelöst durch eine Vorrichtung zur Isolation von Parasiten aus organischem Gewebe mit einer luftdicht verschließbaren Kammer, in der eine Rotationsvorrichtung eingebracht ist, die ausgebildet ist, ein in der Kammer eingebrachtes organisches Gewebe zu zerkleinern und zu vermengen, wobei eine erste Zuführvorrichtung, die als Wasserzuführvorrichtung ausgebildet ist, zur automatischen Zufuhr von Wasser einer vorgebbaren Menge in die Kammer vorgesehen ist, wobei eine zweite Zuführvorrichtung, die als Verdauungspräparatzuführvorrichtung ausgebildet ist, zur automatischen Zufuhr eines Verdauungspräparats einer vorgebbaren Menge in die Kammer vorgesehen ist, und wobei eine Steuervorrichtung vorgesehen ist, die ausgebildet ist, die Rotationsvorrichtung, die erste Zuführvorrichtung und die zweite Zuführvorrichtung zu steuern, wobei die Rotationsvorrichtung eine Rührvorrichtung umfasst, die erste und zweite Rührarme aufweist, wobei wenigstens ein erster Rührarm vorgesehen ist, der längserstreckt ist und in einem Winkel von +30° bis -30° zu einer Senkrechten einer Rotationsachse der Rotationsvorrichtung abgewinkelt ist, wobei wenigstens ein erster Rührarm nach oben und wenigstens ein erster Rührarm nach unten abgewinkelt sind. Erfindungsgemäß ist erkannt worden, dass eine Vorrichtung aus dem Stand der Technik dadurch zu einer schnelleren Isolation von Parasiten aus organischem Gewebe genutzt werden kann, wenn nicht nur eine automatische Zufuhr vorbestimmter oder vorgebbarer Mengen von Wasser und einem Verdauungspräparat in eine luftdicht verschließbare Kammer ermöglicht ist, sondern wenn auch zusätzlich die Steuerung der vorgesehenen Rotationsvorrichtung und der Zuführrichtungen durch eine Steuervorrichtung ermöglicht ist.

Besonders effizient und schnell ist das mit der Vorrichtung durchzuführende Verfahren zur Isolation von Parasiten aus organischem Gewebe, wenn die erste Zuführvorrichtung einen Temperatursensor und eine Wassererwärmvorrichtung umfasst, wobei insbesondere eine Ventilvorrichtung vorgesehen ist, mittels der Wasser beim Fördern von einem Wasservorrat entweder abgelassen oder in die Kammer eingelassen wird. Vorzugsweise steuert die Steuervorrichtung die erste Zuführvorrichtung so, dass erst bei Erreichen einer vorgebbaren Wassertemperatur Wasser in die Kammer eingelassen wird.

Hierdurch wird erwärmtes Wasser, das schon die für die künstliche Verdauung nötige oder bevorzugte Temperatur erreicht hat, in die Kammer eingelassen, so dass sofort die künstliche Verdauung beginnen kann und nicht erst umständlich eine Erwärmung der Rezipienten in der Kammer notwendig ist.

Üblicherweise wird von Hand das zu untersuchende Gewebe in die Kammer eingebracht, dann die Kammer, insbesondere mit einem Bajonettdeckel, der insbesondere aus Edelstahl ist und vorzugweise einen, vorzugsweise abnehmbaren, Öffner- oder Schließhebel aufweist, luftdicht verschlossen und dann entsprechend das Wasser und auch das Verdauungspräparat eingelassen. Wenn das Wasser auf der vorgebbaren Temperatur erwärmt eingelassen wird, ist eine sehr schnelle Verdauung möglich. Hierbei wird vorzugsweise gleichzeitig das erwärmte Wasser in die Kammer eingelassen und das organische Gewebe zerkleinert bzw. wenigstens anschließend auch vermengt. Der Temperatursensor, der ausgangsseitig der Wassererwärmvorrichtung vorgesehen ist, sorgt dafür, dass in dem Moment, wenn die gewünschte Temperatur erreicht ist, Wasser der gewünschten Temperatur auch in die Kammer eingelassen werden kann. Wenn die gewünschte Temperatur nicht erreicht ist, wird das Wasser so lange abgelassen, bis die gewünschte Temperatur vorhanden ist. Die erste Zuführvorrichtung weist als Wassererwärmvorrichtung vorzugsweise auch einen Wassererhitzer und/oder einen Wasserspeicher auf, wobei der Speicher auch als Wärmespeicher ausgebildet sein kann. Zudem kann eine Temperaturregelung vorgesehen sein, um auch die gewünschte Temperatur möglichst exakt einzuhalten, wenn Wasser in die Kammer eingelassen wird.

Vorzugsweise ist zudem auch ein Sensor, beispielsweise ein Durchflusssensor, vorgesehen, der die in die Kammer eingelassene Menge an Wasser misst, so dass in dem Moment, wenn die vorgebbare Menge eingelassen wurde, ein Ventil geschlossen werden kann. Dies kann bevorzugterweise auch über die Steuervorrichtung gesteuert werden.

Die zweite Zuführvorrichtung weist vorzugsweise einen Behälter auf, in dem ein Enzym gelagert ist. Für den Fall, dass das Enzym Pepsin oder ein alternatives Enzym, das in saurer bis neutraler Umgebung für eine Verdauung sorgt, aufgenommen ist, kann vorzugsweise die zweite Zuführvorrichtung auch noch einen weiteren Behälter zur Aufnahme von Salzsäure aufweisen. Es sind auch entsprechende Pumpen und Leitungen in der zweiten Zuführvorrichtung vorgesehen, die eine Zufuhr des Enzyms und gegebenenfalls der Salzsäure in die Kammer ermöglichen.

Vorzugsweise ist ein Sensor vorgesehen, der die Menge des in die Kammer eingelassenen Wassers misst. Der Sensor erzeugt dann ein Messsignal, das an die Steuervorrichtung geleitet wird, um die Wasserzufuhr zu stoppen, wenn eine vorgebbare Menge Wasser in die Kammer eingelassen wurde. Die Ventilvorrichtung kann dann ein Ventil vorsehen, das gesperrt wird, um die weitere Wasserzufuhr in die Kammer zu stoppen.

Vorzugsweise weist die zweite Zuführvorrichtung einen ersten Behälter zur Aufnahme des Verdauungspräparats auf. Zudem weist die zweite Zuführvorrichtung vorzugsweise eine erste Pumpe und entsprechende Leitungen sowie eine Dosiervorrichtung auf, um eine vorgebbare Menge des Verdauungspräparats in die Kammer einlassen zu können. Vorzugsweise ist eine Pumpvorrichtung vorgesehen, mittels der das Verdauungspräparat dosiert in die Kammer einbringbar ist.

Vorzugsweise weist die zweite Zuführvorrichtung einen zweiten Behälter zur Aufnahme von Salzsäure oder einer anderen Säure auf, und insbesondere vorzugsweise auch eine zweite Pumpe, entsprechende Leitungen und eine weitere Dosiervorrichtung, mittels der auch eine vorgebbare Menge an Salzsäure oder einer anderen Säure oder möglicherweise auch einer Base in die Kammer eingebracht werden kann.

Erfindungsgemäß umfasst die Rotationsvorrichtung eine Rührvorrichtung, die erste und zweite Arme bzw. Rührarme aufweist, wobei wenigstens ein erster Rührarm vorgesehen ist, der längserstreckt ist und in einem Winkel von + 30° bis - 30° zu einer Senkrechten einer Rotationsachse der Rotationsvorrichtung abgewinkelt ist. Der Winkel + 30° bis - 30° ist vorzugsweise relativ zur Waagerechten, und zwar insbesondere dann, wenn die Rotationsachse der Rotationsvorrichtung horizontal ausgerichtet ist.

Insbesondere vorzugsweise ist der Winkel zwischen + 20° und - 20° und weiter vorzugsweise zwischen + 10° und - 10°. Hierdurch ist ein effizientes Zerkleinern und Durchmischen des Inhalts der Kammer, insbesondere des Gewebes, möglich.

Vorzugsweise ist wenigstens ein zweiter Rührarm vorgesehen, der wenigstens teilweise mit einem Winkel von 60° bis 120°, insbesondere 70° bis 90°, nach unten zu einem Kammerboden der Kammer abgewinkelt ist. Durch diesen wenigstens einen zweiten Rührarm wird Material, das sich sonst am Kammerboden anlagern würde, auch weiter durchmischt.

Erfindungsgemäß sind wenigstens ein erster Rührarm nach oben und wenigstens ein erster Rührarm nach unten abgewinkelt. Insbesondere vorzugsweise sind wenigstens vier erste Rührarme vorgesehen, wobei jeweils zwei erste Rührarme konzentrisch zueinander ausgerichtet sind und entsprechend gleiche Massen aufweisen bzw. gleiche Längen aufweisen, so dass Unwuchten vermieden werden. Durch die bevorzugte Ausgestaltung, dass wenigstens ein erster Rührarm nach oben und wenigstens ein erster Rührarm nach unten abgewinkelt ist, kann mehr Volumen beim Zerkleinern und Vermengen in der Kammer erfasst werden, so dass eine sehr effiziente Zerkleinerung und Vermengung möglich ist.

Vorzugsweise ist ein steuerbarer Motor vorgesehen, um die Rotationsvorrichtung anzutreiben. Der Motor kann in wenigstens zwei vorgebbaren Geschwindigkeiten betrieben werden, beispielsweise eine Geschwindigkeit, mittels der die Rührvorrichtung der Rotationsvorrichtung für ein Zerkleinern des Gewebes sorgt, und eine Geschwindigkeit, in der kein Zerkleinern mehr stattfindet, sondern nur noch ein Vermengen des zerkleinerten Materials. Der Motor kann auch über ein Getriebe mit der Rotationsvorrichtung verbunden sein bzw. die Rotationsvorrichtung kann ein Getriebe umfassen, so dass der Motor bei gleichbleibender Geschwindigkeit betrieben wird und beispielsweise verschiedene Gänge im Getriebe verwendet werden, um die Rührvorrichtung mit unterschiedlichen Drehgeschwindigkeiten anzutreiben.

Die Rührarme der Rührvorrichtung sind wenigstens teilweise längserstreckt, haben also eine Längserstreckung, die beispielsweise mehr als fünfmal zu groß ist wie die Tiefe der Rührarme bei einer entsprechend dünnen Ausgestaltung. Die Rührarme können beispielsweise eine Dicke von 1 mm oder 2 mm aufweisen und vorzugsweise ungeschärft sein bzw. bereichsweise ungeschärft sein, eine Tiefe von 0,5 bis 2 cm, bevorzugt 1 cm, haben und eine Länge von 40 bis 200 mm aufweisen. Die Länge der Rührarme ist vorzugsweise angepasst an den Innenraum der Kammer. Beispielsweise überstreichen zumindest die längserstreckten Rührarme, die als erste Rührarme bezeichnet sind, über 90% des Durchmessers des Innenraums der Kammer.

Wenigstens ein erster Rührarm ist hierbei vorzugsweise zwei- bis fünfmal so lang wie ein zweiter Rührarm. Der zweite Rührarm ist vorzugsweise nach unten gebogen, beispielsweise zwischen 60° und 90°, und weist zum Boden hin eine Rückbiegung auf, so dass dieser Rührarm L-förmig ist. Die Länge zur Tiefe der ersten Rührarme ist vorzugsweise im Bereich von 20:1 bis 20:4. Der Durchmesser von sich jeweils gegenüberliegenden Rührarmen ist im Verhältnis zum Durchmesser der Kammer, und zwar bei den längserstreckten Rührarmen, d.h. den ersten Rührarmen, bis zu 98% des Durchmessers der Kammer.

Die Drehzahl der Rührvorrichtung liegt bevorzugterweise zwischen 120 und 9.500 U/min, insbesondere vorzugsweise zwischen 250 und 2.500 U/min. Eine bevorzugte Umfangsgeschwindigkeit des Umfangs der ersten Rührarme liegt bei 1,0 bis 20 m/s, insbesondere vorzugsweise bei 2,0 bis 13,8 m/s, bei einem Durchmesser von 170 mm der Rührvorrichtung bzw. der ersten Rührarme der Rührvorrichtung. Die Kammer hat vorzugsweise einen Durchmesser von 80 mm bis 400 mm, insbesondere vorzugsweise zwischen 120 mm und 300 mm, insbesondere vorzugsweise zwischen 150 und 250 mm. Die Kammer gemäß den folgenden Ausführungsbeispielen hat einen Durchmesser von 200 mm. Vorzugsweise ist in dem Innenraum der Kammer, vorzugsweise an der Innenwand, ein Strömungsunterbrecher vorgesehen, mittels dessen das Gewebe bei hoher Drehzahl immer wieder zu den Rührarmen der Rührvorrichtung geführt wird, anstelle dessen, dass das Gewebe nur eine Rotation an der Innenwand ausführen würde.

Die Höhe der Kammer liegt vorzugsweise zwischen 100 mm und 600 mm, insbesondere vorzugsweise zwischen 140 mm und 400 mm, wobei die Kammer der Ausführungsbeispiele eine Höhe von 150 mm hat.

Vorzugsweise wird die Rührvorrichtung im Betrieb mit der Vorrichtung zur Isolation von Parasiten aus organischem Gewebe mit zwei vorgebbaren Drehzahlen betrieben. Hierbei liegt die erste Drehzahl zwischen 1.000 und 9.500 U/min und die zweite Drehzahl zwischen 120 und 300 U/min. Bei einer Drehzahl von 1.000 U/min und einem Durchmesser der ersten Rührarme von 200 mm bzw. fast 200 mm, ergibt sich somit eine Umfangsgeschwindigkeit von ca. 10 m/s. Bei einer Drehzahl von 9.500 U/min ist die Umfangsgeschwindigkeit dann ca. 99 m/s. Ein bevorzugter Wert ist eine erste Drehzahl zwischen 1.000 und 2.000 U/min. Die zweite Drehzahl liegt bei 120 bis 300 U/min, was bei einem Durchmesser von 200 mm im unteren Bereich einer Drehzahl von 1,2 m/s entspricht, und im oberen Bereich bei 3 m/s. Die erste Drehzahl dient insbesondere zum Zerkleinern des organischen Gewebes und die untere bzw. zweite Drehzahl ausschließlich zum Vermengen des organischen Gewebes.

Vorzugsweise ist die Kammer aus Edelstahl oder zumindest ist der Innenraum der Kammer aus Edelstahl, wobei das Edelstahl an einer Innenwand der Kammer oberflächenbehandelt, insbesondere poliert, insbesondere elektropoliert, ist. Hierdurch ist eine sehr effiziente Reinigung der Kammer möglich, so dass eine schnelle Verfahrensführung insgesamt möglich ist. Insbesondere ist hierdurch auch die erfindungsgemäße Vorrichtung sehr langlebig, was mit einer Beschichtung versehene Kammern nicht notwendigerweise sind.

Vorzugsweise weist die Kammer einen Kammerboden auf, der ein Gefälle nach unten zu einer Auslassöffnung aufweist, wobei sich an die Auslassöffnung insbesondere eine Filtervorrichtung anschließt. Durch das Gefälle wird sichergestellt, dass sämtliches verdautes Material abgeführt werden kann. Eine sich an die Auslassöffnung anschließende Filtervorrichtung dient dazu, die Parasiten aufzufangen, wohingegen das verdaute Material und die Flüssigkeiten durch den Filter hindurchgehen sollen.

Vorzugweise weist die Kammer einen Deckel auf, der bajonettartig verschließbar ist. Insbesondere weist die Kammer einen Bajonettdeckel auf, mittels dessen die Kammer verschließbar ist. Vorzugsweise ist zum Verschließen, insbesondere zum Verschließen der Kammer mit dem Bajonettdeckel, ein abnehmbarer Öffnerhebel oder Schließhebel vorgesehen.

Vorzugsweise ist in einem Innenraum der Kammer ein Strömungsunterbrecher vorgesehen. Bei dem Strömungsunterbrecher kann es sich um eine Wulst an der Innenwand der Kammer handeln oder mehrere Wülste, die bei einer Strömung entlang der Kammerinnenwand dem dort strömenden Medium eine Bewegungskomponente nach innen in die Kammer geben.

Die Aufgabe wird ferner durch ein Verfahren zum Betreiben einer Vorrichtung zur Isolation von Parasiten aus organischem Gewebe, gelöst, die vorzugsweise die oben genannten Merkmale aufweist, wobei die folgenden Verfahrensschritte vorgesehen sind:
- Einbringen einer vorgebbaren Menge an organischem Gewebe in eine Kammer,
- Einbringen einer vorgebbaren Menge von auf eine vorbestimmte Temperatur erwärmten Wassers in die Kammer,
- Einbringen eines Verdauungspräparats, insbesondere eines Verdauungsenzyms, einer vorgebbaren Menge in die Kammer,
- Zerkleinern des organischen Gewebes mittels einer Rührvorrichtung, die zum Zerkleinern des organischen Gewebes mit einer ersten Drehzahl betrieben wird,
- Rühren des Inhalts der Kammer mittels der Rührvorrichtung mit einer zweiten Drehzahl, wobei die zweite Drehzahl kleiner als die erste Drehzahl ist,
- Öffnen eines Ablassventils und Beaufschlagen des Inneren der Kammer mit einem Überdruck, so dass der Inhalt der Kammer durch eine an einem Ausgang der Kammer vorgesehenen Filtervorrichtung geführt wird.

Vorzugsweise wird ergänzend zu dem Einbringen des Verdauungspräparats, insbesondere Verdauungsenzyms, eine Säure, insbesondere Salzsäure, in die Kammer eingeleitet.

Vorzugsweise ist eine Temperaturregelung des Inhalts der Kammer auf die vorbestimmte Temperatur vorgesehen. Bei der vorbestimmten Temperatur handelt es sich vorzugsweise um die Temperatur, die für das einzulassende Wasser vorbestimmt ist, wobei es sich vorzugsweise um eine auf das Verdauungspräparat und das Gewebe für die Verdauung optimierte Temperatur handelt. Hierbei ist insbesondere ein Temperatursensor in der Kammer bzw. in einer Wand der Kammer vorgesehen.

Der Temperatursensor ist vorzugsweise mit einer Steuervorrichtung oder einer Regelvorrichtung verbunden, die eine Heizvorrichtung, beispielsweise eine um die Kammer angebrachte Heizfolie, steuert. Hierdurch kann die in der Kammer herrschende Temperatur geregelt werden. Alternativ zu einer Heizfolie oder einer Heizmatte, die um die Kammer gelegt wird, kann die Wand der Kammer auch mit einem Leitungssystem versehen werden, durch das auf die vorbestimmte Temperatur gebrachtes Wasser geführt wird, um die Kammerwände auf einer vorbestimmten Temperatur zu halten.

Die Temperaturdifferenz bzw. -varianz zu der vorbestimmten oder gewählten Temperatur kann je nach Regelgenauigkeit eingestellt werden. Vorzugsweise wird die Temperatur in einem Bereich von weniger als 10 °C, insbesondere vorzugsweise weniger als 5 °C, insbesondere vorzugsweise weniger als 2 °C, konstant gehalten.

Vorzugsweise liegt die erste Drehzahl zwischen 1.000 und 9.500 U/min und/oder die zweite Drehzahl zwischen 120 und 300 U/min.

Vorzugsweise ist zum Nachweis von Trichinen in Fleisch und bei einem Verdauungsenzym, das eine Serin-Endopeptidase ist, eine vorgebbare Temperatur von 55 °C bis 65 °C vorgesehen, oder zum Nachweis von Trichinen in Fleisch und bei einem Verdauungsenzym, das Pepsin ist, eine vorgebbare Temperatur von 45 °C bis 55 °C vorgesehen, oder zum Nachweis von Nematoden in Fisch bei einem Verdauungsenzym, das Pepsin ist, eine vorgebbare Temperatur von 30 °C bis 40 °C vorgesehen.

Vorzugsweise ist eine Probenmenge an organischem Gewebe in die Kammer einbringbar, die einem Gewicht von 50 g bis 200 g entspricht. Insbesondere vorzugsweise liegt das Gewicht bei 100 g. Hierdurch können 100 Proben à 1 g eingebracht werden, so dass beispielsweise 100 Schweine auf Trichinen untersucht werden können.

Bei der Ausführung des Verfahrens wird Leitungswasser beispielsweise in einem Wärmespeicher auf 60 °C Bezugstemperatur gebracht und in dem Wärmespeicher bevorratet. Zum Start einer Messung bzw. zum Start der Isolation von Parasiten in einem organischen Gewebe wird auf 60 °C erwärmtes Wasser in eine Kammer, insbesondere Reaktionskammer, eingebracht, nachdem das Gewebe in die Kammer eingebracht worden ist. Solange das Wasser noch keine Temperatur von 60 °C erreicht hat, wird das Wasser aus der Zuleitung zwischen dem Wärmespeicher und der Kammer abgelassen. Sobald ein Temperatursensor, der in der Zuleitung angeordnet ist, 60 °C misst, wird das Wasser in die Kammer eingelassen. Zeitgleich oder kurz danach fördern entsprechende Pumpen weitere Komponenten, wie ein Verdauungsenzym und gegebenenfalls eine Säure, in die Kammer hinein.

Es wird dann über eine Ansteuerung eines Motors eine Rührvorrichtung angetrieben. Dieses kann parallel zum Einlassen des Wassers und/oder der weiteren Komponenten geschehen oder kurz nachdem diese Komponenten eingebracht worden sind. Der Motor bzw. die Rührvorrichtung wird mit zwei unterschiedlichen Drehzahlen betrieben. Die erste Drehzahl, die beispielsweise über drei Minuten angesteuert wird, ist eine Drehzahl, bei der das organische Material zerkleinert wird. Die zweite Drehzahl, die während des gesamten Verdauungsprozesses nach dem Zerkleinern verwendet wird, dient ausschließlich zum Rühren des organischen Materials mit den weiteren in die Kammer eingebrachten Komponenten. Nach einer vorgebbaren Zeit, beispielsweise 30 Minuten, ist die Verdauung beendet und die Kammer wird unter Druck gesetzt, wobei ein Auslassventil geöffnet wird und das in der Kammer befindliche Material durch den Auslass bzw. das Auslassventil über eine Membran oder einen Filter, der in einem Filtergehäuse angeordnet ist, abgelassen. Das organische Material mit Ausnahme der Parasiten ist so klein und soweit zerkleinert und verdaut, dass dieses durch die Filtermembran, den Filter oder die Membran hindurchtritt. Die Parasiten bleiben allerdings in dem Filter bzw. auf dem Filter hängen und können dann beispielsweise durch optische oder mikroskopische Untersuchungen festgestellt werden.

Weitere Merkmale der Erfindung werden aus der Beschreibung erfindungsgemäßer Ausführungsformen zusammen mit den Ansprüchen und den beigefügten Zeichnungen ersichtlich. Erfindungsgemäße Ausführungsformen können einzelne Merkmale oder eine Kombination mehrerer Merkmale erfüllen.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:
- Fig. 1: eine schematische Darstellung einer erfindungsgemäßen Vorrichtung zur Isolation von Parasiten aus organischem Gewebe,
- Fig. 2: eine schematische dreidimensionale Darstellung einer für die Erfindung verwendete Rührvorrichtung und
- Fig. 3: eine schematische Darstellung einer weiteren erfindungsgemäßen Vorrichtung.

In den Zeichnungen sind jeweils gleiche oder gleichartige Elemente und/oder Teile mit denselben Bezugsziffern versehen, so dass von einer erneuten Vorstellung jeweils abgesehen wird.

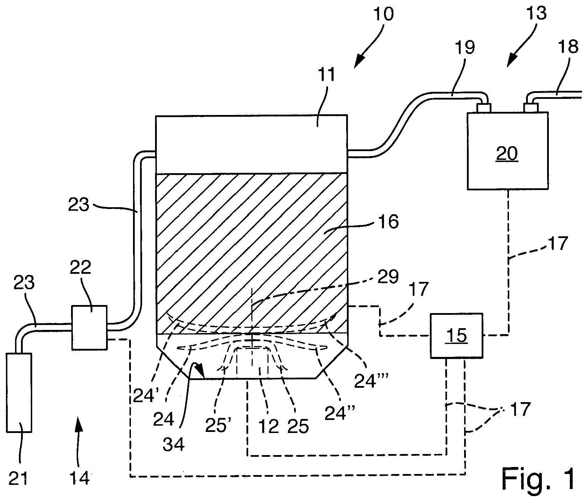

Fig. 1 zeigt schematisch eine erfindungsgemäße Vorrichtung zur Isolation von Parasiten aus organischem Gewebe, die im Folgenden Isolationsvorrichtung 10 genannt wird. Die Isolationsvorrichtung 10 weist eine Kammer 11 auf, in die organisches Gewebe beispielsweise von oben durch eine nicht dargestellte Öffnung eingebracht werden kann. Die Öffnung wird nach dem Einbringen des organischen Gewebes geschlossen, insbesondere luftdicht geschlossen.

Um die Kammer 11 herum ist eine Heizfolie 16 angeordnet, um die Kammer 11 auf einer vorbestimmten Temperatur zu halten. Nach dem Einbringen des organischen Gewebes wird Wasser über einen Wasseranschluss 18 und einen Durchlauferhitzer 20 sowie eine Wasserleitung 19, die Bestandteil einer ersten Zuführvorrichtung 13 sind, auf eine vorbestimmte Temperatur vorgewärmt und in die Kammer 11 eingelassen.

Zudem wird zeitgleich oder kurz danach ein Enzym bzw. ein Verdauungspräparat aus einem Enzymbehälter 21 über eine Leitung 23 und mit Hilfe einer Förderpumpe 22 auch in die Kammer 11 eingelassen. Dieses geschieht automatisch und gesteuert über eine Steuervorrichtung 15. Die Steuervorrichtung 15 ist über elektronische Leitungen 17 mit entsprechenden Komponenten der ersten Zuführvorrichtung 13 und der zweiten Zuführvorrichtung 14 verbunden. Die zweite Zuführvorrichtung 14 umfasst den Enzymbehälter 21, die Leitung 23 und die Förderpumpe 22.

Die Steuervorrichtung 15 steuert auch über eine elektronische Leitung 17 die Heizfolie 16 an. Zudem steuert die Steuervorrichtung 15 die Rührvorrichtung 12, und zwar dergestalt, dass diese rotierend angetrieben wird, beispielsweise durch einen in Fig. 1 nicht dargestellten Motor.

Die Rührvorrichtung 12 weist erste Rührarme 24 bis 24'" auf, wobei die Rührarme 24' und 24'" sich im Wesentlichen über die gesamte Kammer 11 erstrecken und über eine Rotationsachse 29 rotierend angetrieben sind. Entsprechend sind auch die Rührarme 24 und 24" längserstreckt. Die Rührarme 24 und 24" auf der einen Seite und 24' und 24'" auf der anderen Seite können einteilig miteinander verbunden sein, allerdings auch aus mehreren Teilen bestehen. Es ist angedeutet, dass die ersten Rührarme 24' und 24'" etwas nach oben gebogen sind und die Rührarme 24 sowie 24" etwas nach unten gebogen sind, um ein größeres Volumen des in die Kammer 11 eingebrachten Materials zu erfassen. Zudem sind die Rührarme 24', 24'" auf der einen Seite und 24, 24" auf der anderen Seite vorzugsweise diametral zueinander angeordnet.

Es sind ferner zweite Rührarme 25 und 25' vorgesehen, die vorzugsweise auch einteilig miteinander verbunden sind, und nach unten zum Kammerboden 34 der Kammer 11 gebogen sind. Es ist auch eine gewisse Rückbiegung zu erkennen, so dass sich eine L-Form dieser Rührarme 25, 25' ergibt. Die zweiten Rührarme 25, 25' dienen dazu zu verhindern, dass sich Material am Kammerboden 34 oder im Bereich des Kammerbodens 34 der Kammer 11 festsetzt.

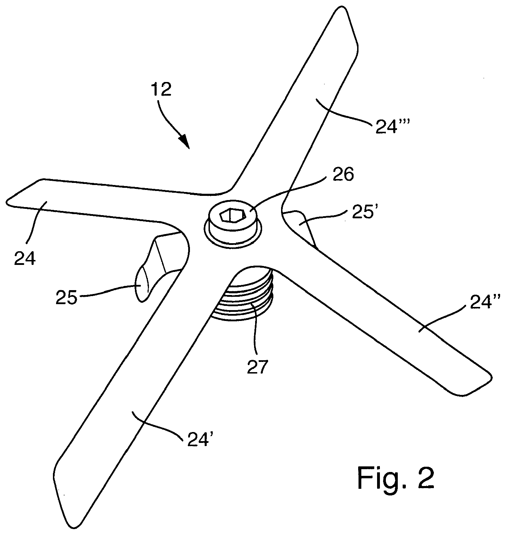

In Fig. 2 ist die Rührvorrichtung 12 schematisch in einer dreidimensionalen Darstellung gezeigt. Es sind die längserstreckten Rührarme 24 bis 24'" dargestellt. Die Rührarme sind einteilig in Form eines vierarmigen Sterns ausgebildet. Die Endbereiche, d.h. die nach außen liegenden Bereiche, sind bei den jeweiligen Rührarmen winkelig ausgebildet bzw. spitz zulaufend. Es sind auch die zweiten Rührarme 25, 25' gezeigt, die nach unten gebogen mit Gegenbiegung etwas L-förmig ausgebildet sind. Die zweiten Rührarme 25, 25' sind vorzugsweise auch einstückig miteinander ausgebildet. Die jeweiligen Rührarme sind über eine Befestigungsschraube 26 mit einer Antriebswelle verbunden und über eine Feder 27 federvorgespannt eingebracht bzw. angebracht, so dass eine stabile Rotation mit möglichst wenig Unwucht möglich ist.

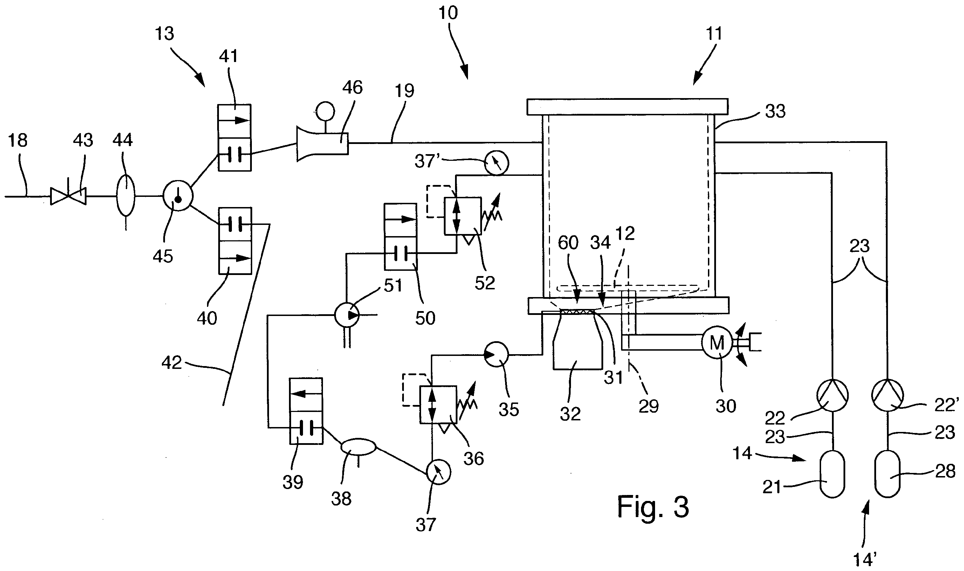

Fig. 3 zeigt schematisch eine weitere erfindungsgemäße Isolationsvorrichtung 10, umfassend eine Kammer 11, in der in Fig. 3 schematisch eine Wand 33 dargestellt ist. Der Kammerboden 34 ist hier schematisch auch angedeutet nach unten geneigt zu einer Auslassöffnung 60 ausgerichtet. Die in der Kammer befindlichen Teile sind gestrichelt dargestellt, um darzustellen, dass diese in einer Ansicht verdeckt wären. Es ist auch eine entsprechende Rührvorrichtung 12 nur schematisch angedeutet. Die Rührvorrichtung 12 rotiert über eine Rotationsachse 26 und ist angetrieben durch einen Motor 30 und ein entsprechendes Umlenkgetriebe. In diesem Ausführungsbeispiel ist ein weiterer Behälter, in diesem Fall ein Säurebehälter 28, der auch ein Basenbehälter sein kann, vorgesehen, wobei die Säure bzw. die Base über eine Leitung 23 und eine Pumpe 22', die als Förderpumpe ausgestaltet ist, mit der Kammer 11 verbunden ist.

Der Säurebehälter 28 mit der Förderpumpe 22' und den entsprechenden zugehörigen Leitungen 23 bilden die zweite Zuführvorrichtung 14'.

Die erste Zuführvorrichtung 13 dient zur Wasserzufuhr und ist in diesem Ausführungsbeispiel wie folgt ausgestaltet. Über einen Wasseranschluss 18 und ein Absperrventil 23 gelangt Wasser in einen Wärmespeicher 44. Der Wärmespeicher 44 erhitzt das Wasser auch auf eine gewünschte Temperatur. Es kann sich hierbei auch um einen Durchlauferhitzer handeln. Anschließend an den Wärmespeicher ist ein Temperatursensor 45 vorgesehen. Der Temperatursensor 45 kann auch unmittelbar vor der Kammer 11 angeordnet sein. In diesem Ausführungsbeispiel der Fig. 3 ist in Förderrichtung des Wassers stromabwärts des Temperatursensors 45 die Wasserleitung in zwei Wege aufgeteilt. Beide Wege führen jeweils zu einem Ventil 40 bzw. 41. Hierbei handelt es sich um 2/2-WegeVentile.

Solange die Wassertemperatur zu niedrig ist, wird das Wasser über die Betätigung des 2/2-Wege-Ventils 40' zur Abwasserleitung 42 geführt. Sobald die vorgegebene Temperatur erreicht ist, wird das 2/2-Wege-Ventil 40 gesperrt und das 2/2-Wege-Ventil 41 geöffnet, so dass Wasser über die Leitung 19 in die Kammer 11 geführt wird. Es ist ein Durchflusssensor 46 vorgesehen, der die Wassermenge misst. Der Durchflusssensor 46 ist mit einer in Fig. 3 nicht dargestellten Steuervorrichtung verbunden und sorgt dafür, dass mit Erreichen der gewünschten Wassermenge, die in die Kammer 11 eingebracht wurde, das Ventil 43 und/oder das Ventil 41 geschlossen wird.

Zudem ist in Fig. 3 ein Kompressor 51 gezeigt, der Druckluft in Richtung des 2/2-Wege-Ventils 50 und auch Richtung des 2/2-Wege-Ventils 39 fördert. Während des Zeitraums der Verdauung füllt der Kompressor 51 den Druckspeicher 38, indem das 2/2-Wege-Ventil 39 geöffnet wird. Sobald ein vorgebbarer Druck erreicht wird, wird dieses Ventil wieder geschlossen. Sobald die Verdauung beendet ist, wird der Kompressor 51 wieder angesteuert und es wird Druckluft über das 2/2-Wege-Ventil 50 in die Kammer 11 eingelassen. Hierdurch wird die Kammer 11 unter Druck gesetzt.

Um zu verhindern, dass dort ein zu hoher Druck entsteht, ist ein Druckregelventil 52, das als Rückströmventil ausgebildet ist, vorgesehen. Es kann auch vorgesehen sein, dass ein Drucksensor die nicht dargestellte Steuervorrichtung mit einem Druckwert versorgt, woraufhin die Steuervorrichtung den Kompressor 51 oder das Ventil 50 abschalten kann. Das Manometer 37' dient zur Überwachung dieses Drucks, beispielsweise für Bedienpersonen. Als Druck, der in die Kammer eingebracht wird, ist ein Druck zwischen 0,05 und 0,2 bar vorzugsweise vorgesehen.

Nachdem die Kammer 11 mit Druck befüllt ist, öffnet ein pneumatischer Drehantrieb 35 ein am Auslass 60 der Kammer 11 angeordnetes Ventil 31, so dass die in der Kammer 11 vorhandene Lösung, insbesondere das verdaute Gewebe, durch das Filtergehäuse 32 geleitet wird, wobei das im Filter nicht zurückgehaltene Material in einen Abfallbehälter geleitet wird. Hier dient der Druckspeicher 38 dazu, den pneumatischen Drehantrieb 35 zu betätigen. Auch wird über ein Druckregelventil 36 dafür gesorgt, dass kein zu hoher Druck am pneumatischen Drehantrieb 35 vorliegt. Es ist zudem auch ein Manometer bzw. ein Druckmessgerät 37 hier zur Kontrolle vorgesehen.

Nachdem das in der Kammer 11 befindliche Material abgelassen wurde, kann aus dem Filtergehäuse 32 der Filter bzw. die Filtermembran entnommen werden und überprüft werden, ob Parasiten im organischen Gewebe vorhanden gewesen sind.

Die erfindungsgemäße Vorrichtung und das erfindungsgemäße Verfahren ermöglichen eine sehr schnelle und automatisierte Isolation von Parasiten aus organischem Gewebe. Zudem sind sehr hohe Mengen an organischem Gewebe verwendbar. Es ist eine sehr verlässliche Isolation von Parasiten aus organischem Gewebe möglich.

Alle genannten Merkmale, auch die den Zeichnungen allein zu entnehmenden sowie auch einzelne Merkmale, die in Kombination mit anderen Merkmalen offenbart sind, werden allein und in Kombination als erfindungswesentlich angesehen. Erfindungsgemäße Ausführungsformen können durch einzelne Merkmale oder eine Kombination mehrerer Merkmale erfüllt sein. Im Rahmen der Erfindung sind Merkmale, die mit "insbesondere" oder "vorzugsweise" gekennzeichnet sind, als fakultative Merkmale zu verstehen.

### Bezugszeichenliste

- 10: Isolationsvorrichtung
- 11: Kammer
- 12: Rührvorrichtung
- 13: erste Zuführvorrichtung
- 14: zweite Zuführvorrichtung
- 15: Steuervorrichtung
- 16: Heizfolie
- 17: elektronische Leitung
- 18: Wasseranschluss
- 19: Wasserleitung
- 20: Durchlauferhitzer
- 21: Enzymbehälter
- 22, 22': Förderpumpe
- 23: Leitung
- 24, 24', 24", 24'": erster Rührarm
- 25, 25': zweiter Rührarm
- 26: Befestigungsschraube
- 27: Feder
- 28: Säurebehälter
- 29: Rotationsachse
- 30: Motor
- 31: Ventil
- 32: Filtergehäuse
- 33: Wand
- 34: Kammerboden
- 35: pneumatischer Drehantrieb
- 36: Druckregelventil
- 37, 37': Druckmessgerät
- 38: Druckspeicher
- 39: 2/2-Wege-Ventil
- 40: 2/2-Wege-Ventil
- 41: 2/2-Wege-Ventil
- 42: Abwasserleitung
- 43: Absperrventil
- 44: Wärmespeicher
- 45: Temperatursensor
- 46: Durchflusssensor
- 50: 2/2-Wege-Ventil
- 51: Kompressor
- 52: Druckregelventil
- 60: Auslassöffnung

## Patentansprüche

1. Vorrichtung (10) zur Isolation von Parasiten aus organischem Gewebe mit einer luftdicht verschließbaren Kammer (11), in der eine Rotationsvorrichtung (12) eingebracht ist, die ausgebildet ist, ein in der Kammer (11) eingebrachtes organisches Gewebe zu zerkleinern und zu vermengen, wobei eine erste Zuführvorrichtung (13), die als Wasserzuführvorrichtung ausgebildet ist, zur automatischen Zufuhr von Wasser einer vorgebbaren Menge in die Kammer (11) vorgesehen ist, wobei eine zweite Zuführvorrichtung (14), die als Verdauungspräparatzuführvorrichtung ausgebildet ist, zur automatischen Zufuhr eines Verdauungspräparats einer vorgebbaren Menge in die Kammer (11) vorgesehen ist, und wobei eine Steuervorrichtung (15) vorgesehen ist, die ausgebildet ist, die Rotationsvorrichtung (12), die erste Zuführvorrichtung (13) und die zweite Zuführvorrichtung (14) zu steuern, wobei die Rotationsvorrichtung (12) eine Rührvorrichtung (12) umfasst, die erste und zweite Rührarme (24, 24', 24", 24"', 25, 25') aufweist, wobei wenigstens ein erster Rührarm (24-24'") vorgesehen ist, der längserstreckt ist und in einem Winkel von + 30° bis - 30° zu einer Senkrechten einer Rotationsachse (29) der Rotationsvorrichtung (12) abgewinkelt ist, wobei wenigstens ein erster Rührarm (24', 24'") nach oben und wenigstens ein erster Rührarm (24, 24") nach unten abgewinkelt sind, wobei wenigstens ein zweiter Rührarm (25, 25') vorgesehen ist, der wenigstens teilweise mit einem Winkel von 60° bis 120°, insbesondere 70° bis 90°, nach unten zu einem Kammerboden (34) der Kammer (11) abgewinkelt ist.

2. Vorrichtung (10) nach Anspruch 1, **dadurch gekennzeichnet, dass** die erste Zuführvorrichtung (13) einen Temperatursensor (45) und eine Wassererwärmvorrichtung (20, 44) umfasst, wobei insbesondere eine Ventilvorrichtung (40, 41) vorgesehen ist, mittels der Wasser beim Fördern von einem Wasservorrat (18, 20, 44) entweder abgelassen wird oder in die Kammer (11) eingelassen wird.

3. Vorrichtung (10) nach Anspruch 1 oder 2, **dadurch gekennzeichnet, dass** ein Sensor (46) vorgesehen ist, der die Menge des in die Kammer (11) eingelassenen Wassers misst.

4. Vorrichtung (10) nach einem der Ansprüche 1 bis 3, **dadurch gekennzeichnet, dass** die zweite Zuführvorrichtung (14) einen ersten Behälter (21) zur Aufnahme des Verdauungspräparats aufweist, wobei insbesondere eine Pumpvorrichtung (22) vorgesehen ist, mittels der das Verdauungspräparat dosiert in die Kammer (11) einbringbar ist.

5. Vorrichtung (10) nach einem der Ansprüche 1 bis 4, **dadurch gekennzeichnet, dass** die Kammer (11) aus Edelstahl ist oder zumindest der Innenraum der Kammer (11) aus Edelstahl ist, wobei das Edelstahl an einer Innenwand (33) der Kammer (11) oberflächenbehandelt, insbesondere poliert, insbesondere elektropoliert, ist.

6. Vorrichtung (10) nach einem der Ansprüche 1 bis 5, **dadurch gekennzeichnet, dass** die Kammer (11) einen Kammerboden (34) aufweist, der ein Gefälle nach unten zu einer Auslassöffnung (60) aufweist, wobei sich an die Auslassöffnung (31) insbesondere eine Filtervorrichtung (32) anschließt, wobei insbesondere die Kammer (11) einen Bajonettdeckel aufweist, mittels dessen die Kammer (11) verschließbar ist, wobei insbesondere zum Verschließen ein abnehmbarer Öffnerhebel oder Schließhebel vorgesehen ist, wobei insbesondere in einem Innenraum der Kammer (11) ein Strömungsunterbrecher vorgesehen ist.

7. Verfahren zum Betreiben einer Vorrichtung (10) zur Isolation von Parasiten aus organischem Gewebe nach einem der Ansprüche 1 bis 6 mit den folgenden Verfahrensschritten:
- Einbringen einer vorgebbaren Menge an organischem Gewebe in eine Kammer (11),
- Einbringen einer vorgebbaren Menge von auf eine vorbestimmte Temperatur erwärmten Wassers in die Kammer (11),
- Einbringen eines Verdauungspräparats, insbesondere eines Verdauungsenzyms, einer vorgebbaren Menge in die Kammer (11),
- Zerkleinern des organischen Gewebes mittels einer Rührvorrichtung (12), die zum Zerkleinern des organischen Gewebes mit einer ersten Drehzahl betrieben wird,
- Rühren des Inhalts der Kammer (11) mittels der Rührvorrichtung (12) mit einer zweiten Drehzahl, wobei die zweite Drehzahl kleiner als die erste Drehzahl ist,
- Öffnen eines Ablassventils (31) und Beaufschlagen des Inneren der Kammer (11) mit einem Überdruck, so dass der Inhalt der Kammer (11) durch eine an einem Ausgang (60) der Kammer (11) vorgesehenen Filtervorrichtung (32) geführt wird.

8. Verfahren nach Anspruch 7, **dadurch gekennzeichnet, dass** ergänzend zu dem Einbringen des Verdauungspräparats eine Säure, insbesondere Salzsäure, in die Kammer (11) eingeleitet wird.

9. Verfahren nach Anspruch 7 oder 8, **dadurch gekennzeichnet, dass** eine Temperaturregelung des Inhalts der Kammer (11) auf die vorbestimmte Temperatur vorgesehen ist.

10. Verfahren nach einem der Ansprüche 7 bis 9, **dadurch gekennzeichnet, dass** die erste Drehzahl zwischen 1.000 und 9.500 U/min liegt und/oder die zweite Drehzahl zwischen 120 und 300 U/min liegt.

11. Verfahren nach einem der Ansprüche 7 bis 10, **dadurch gekennzeichnet, dass** zum Nachweis von Trichinen in Fleisch und bei einem Verdauungsenzym, das eine Serin-Endopeptidase ist, eine vorgebbare Temperatur von 55 °C bis 65 °C vorgesehen ist, oder bei einem Verdauungsenzym, das Pepsin ist, eine vorgebbare Temperatur von 45 °C bis 55 °C vorgesehen ist, oder dass zum Nachweis von Nematoden in Fisch bei einem Verdauungsenzym, das Pepsin ist, eine vorgebbare Temperatur von 30 °C bis 40 °C vorgesehen ist.

## Claims

1. An apparatus (10) to isolate parasites from organic tissue with a chamber (11) that can be closed airtight and into which a rotation apparatus (12) is introduced which is designed to comminute and mix an organic tissue introduced into the chamber (11), wherein a first supply apparatus (13) that is designed as a water supply apparatus is provided to automatically supply water at a specifiable amount to the chamber (11), wherein a second supply apparatus (14) that is designed as a digestive preparation supply apparatus is provided to automatically supply a digestive preparation at a specifiable amount to the chamber (11), and wherein a control apparatus (15) is provided that is designed to control the rotation apparatus (12), the first supply apparatus (13) and the second supply apparatus (14), wherein the rotation apparatus (12) comprises a stirring apparatus (12) that has first and second stirring arms (24, 24', 24", 24"', 25, 25'), wherein at least a first stirring arm (24-24'") is provided that is elongated and is angled at an angle of +30° to -30° to a perpendicular of a rotational axis (29) of the rotation apparatus (12), wherein at least one first steering arm (24', 24'") is angled upward, and at least one first stirring arm (24, 24") is angled downward, wherein at least one second stirring arm (25, 25') is provided that is at least partially angled downward at an angle of 60° to 120°, in particular 70° to 90°, toward a chamber floor (34) of the chamber (11).

2. The apparatus (10) according to claim 1, **characterized in that** the first supply apparatus (13) comprises a temperature sensor (45) and a water heating apparatus (20, 44), wherein in particular a valve apparatus (40, 41) is provided by means of which water, when delivered from a water reservoir (18, 20, 44), is either drained or fed into the chamber (11).

3. The apparatus (10) according to claim 1 or 2, **characterized in that** a sensor (46) is provided that measures the amount of water fed into the chamber (11).

4. The apparatus (10) according to one of claims 1 to 3, **characterized in that** the second supply apparatus (14) has a first container (21) for receiving the digestive preparation, wherein in particular a pump apparatus (22) is provided by means of which the digestive preparation can be fed into the chamber (11) in a metered manner.

5. The apparatus (10) according to one of claims 1 to 4, **characterized in that** the chamber (11) consists of stainless steel, or at least the interior of the chamber (11) consists of stainless steel, wherein the stainless steel is surface-treated, in particular polished, in particular electropolished, on an inner wall (33) of the chamber (11).

6. The apparatus (10) according to one of claims 1 to 5, **characterized in that** the chamber (11) has a chamber floor (34) that has a downward gradient toward an outlet opening (60), wherein in particular a filter apparatus (32) is connected to the outlet opening (31), wherein in particular the chamber (11) has a bayonet cover by means of which the chamber (11) can be closed, wherein in particular a removable opening lever or closing lever is provided for closing, wherein in particular a flow interrupter is provided in an interior of the chamber (11).

7. The method for operating an apparatus (10) for isolating parasites from organic tissue according to one of claims 1 to 6 having the following method steps:
- feed a specifiable amount of organic tissue into a chamber (11),
- feed a specifiable amount of water heated to a predetermined temperature into the chamber (11),
- feed a digestive preparation, in particular a digestive enzyme, at a specifiable amount into the chamber (11),
- comminute the organic tissue by means of a stirring apparatus (12) that is operated to comminute the organic tissue at a first rotational speed,
- stir the contents of the chamber (11) by means of the stirring apparatus (12) at a second rotational speed, wherein the second rotational speed is slower than the first rotational speed,
- open a drain valve (31) and apply an overpressure to the interior of the chamber (11) so that the contents of the chamber (11) are guided through a filter apparatus (32) provided at an outlet (60) of the chamber (11).

8. A method according to claim 7, **characterized in that** in addition to feeding the digestive preparation, an acid, in particular HCl, is introduced into the chamber (11).

9. The method according to claim 7 or 8, **characterized in that** a temperature regulation of the content of the chamber (11) to the predetermined temperature is provided.

10. The method according to one of claims 7 to 9, **characterized in that** the first rotational speed lies between 1,000 and 9,500 RPM, and/or the second rotational speed lies between 120 and 300 RPM.

11. The method according to one of claims 7 to 10, **characterized in that** to demonstrate trichinella in meat and when there is a digestive enzyme that is a serine endopeptidase, a specifiable temperature of 55°C to 65°C is provided, or when there is a digestive enzyme that is pepsine, a specifiable temperature of 45°C to 55°C is provided, or to demonstrate nematodes in fish when there is a digestive enzyme that is pepsin, a specifiable temperature of 30°C to 40°C is provided.

## Revendications

1. Dispositif (10) pour isoler un tissu organique vis-à-vis de parasites avec une chambre (11) apte à être fermée hermétiquement, dans laquelle est placé un dispositif de rotation (12) conçu pour fragmenter et mélanger un tissu organique introduit dans la chambre (11), un premier dispositif d'alimentation (13) étant conçu comme un dispositif d'alimentation en eau, pour l'alimentation automatique en eau dans la chambre (11) en une quantité prédéterminée, un deuxième dispositif d'alimentation (14), qui est conçu comme un dispositif d'alimentation en préparation digestive, étant prévu pour l'alimentation automatique en une préparation digestive dans la chambre (11) selon une quantité prédéterminée, et un dispositif de commande (15) étant prévu, qui est conçu pour commander le dispositif de rotation (12), ledit premier dispositif d'alimentation (13) et ledit deuxième dispositif d'alimentation (14), ledit dispositif de rotation (12) comprenant un dispositif d'agitation (12) ayant des premier et deuxième bras d'agitation (24, 24', 24", 24"', 25, 25'), au moins un premier bras d'agitation (24-24"') étant prévu de façon à être allongé et incliné selon un angle compris entre +30° et -30° par rapport à une perpendiculaire à un axe de rotation (29) dudit dispositif de rotation (12), au moins un premier bras d'agitation (24', 24"") étant incliné vers le haut et au moins un premier bras d'agitation (24, 24") étant incliné vers le bas, au moins un deuxième bras d'agitation (25, 25') étant prévu en étant au moins partiellement incliné vers le bas selon un angle de 60° à 120°, notamment 70° à 90°, vers un fond (34) de la chambre (11).

2. Dispositif (10) selon la revendication 1, **caractérisé en ce que** le premier dispositif d'alimentation (13) comprend un capteur de température (45) et un dispositif de chauffage de l'eau (20, 44), un dispositif formant vanne (40, 41) étant notamment prévu, au moyen duquel de l'eau est soit évacuée soit introduite dans la chambre (11) depuis un réservoir d'eau (18, 20, 44).

3. Dispositif (10) selon la revendication 1 ou la revendication 2, **caractérisé en ce qu'**il est prévu un capteur (46) qui mesure la quantité d'eau introduite dans la chambre (11).

4. Dispositif (10) selon l'une des revendications 1 à 3, **caractérisé en ce que** le deuxième dispositif d'alimentation (14) présente un premier récipient (21) pour recevoir la préparation digestive, en particulier **en ce qu'**un dispositif de pompage (22) est prévu, au moyen duquel la préparation digestive peut être introduite dans la chambre (11) en quantités dosées.

5. Dispositif (10) selon l'une des revendications 1 à 4, **caractérisé en ce que** la chambre (11) est en acier inoxydable ou au moins l'intérieur de la chambre (11) est en acier inoxydable, l'acier inoxydable étant traité en surface sur une paroi intérieure (33) de la chambre (11), notamment poli, en particulier électro-poli.

6. Dispositif (10) selon l'une des revendications 1 à 5, **caractérisé en ce que** la chambre (11) présente un fond de chambre (34) qui présente une pente descendante vers une ouverture de sortie (60), laquelle est reliée à l'ouverture de sortie (31) notamment par un dispositif de filtrage (32), la chambre (11) présentant notamment un couvercle à baïonnette au moyen duquel la chambre (11) est apte à être fermée, un levier amovible d'ouverture ou de fermeture étant prévu notamment pour la fermeture, un interrupteur de flux étant notamment prévu dans une zone intérieure de la chambre (11).

7. Procédé de fonctionnement d'un dispositif (10) pour isoler des tissus organiques vis-à-vis des parasites selon l'une des revendications 1 à 6 ayant les étapes de procédé suivantes :
- introduction d'une quantité prédéterminée de tissu organique dans une chambre (11),
- introduction d'une quantité prédéterminée d'eau chauffée à une température prédéterminée dans la chambre (11),
- introduction d'une préparation digestive, en particulier une enzyme digestive, en une quantité prédéterminée dans la chambre (11),
- fragmentation du tissu organique au moyen d'un dispositif agitateur (12) qui est utilisé à une première vitesse de rotation pour fragmenter le tissu organique,
- agitation du contenu de la chambre (11) au moyen du dispositif d'agitation (12) à une deuxième vitesse, la deuxième vitesse étant inférieure à la première vitesse,
- ouverture d'une vanne de vidange (31) et mise sous pression de l'intérieur de la chambre (11) de sorte que le contenu de la chambre (11) passe par un dispositif filtrant (32) prévu à une sortie (60) de la chambre (11).

8. Procédé selon la revendication 7, **caractérisé en ce que**, outre l'introduction de la préparation digestive, est introduit dans la chambre (11) un acide, en particulier de l'acide chlorhydrique.

9. Procédé selon la revendication 7 ou la revendication 8, **caractérisé en ce qu'**il est prévu une régulation de température du contenu de la chambre (11) à la température prédéterminée.

10. Procédé selon l'une des revendications 7 à 9, **caractérisé en ce que** la première vitesse est comprise entre 1000 et 9500 tr/min et/ou **en ce que** la deuxième vitesse est comprise entre 120 et 300 tr/min.

11. Procédé selon l'une quelconque des revendications 7 à 10, **caractérisé en ce que**, pour détecter des trichines dans de la viande et dans le cas d'une enzyme digestive qui est une sérine endopeptidase, il est prévu une température prédéterminée de 55°C à 65°C, ou, dans le cas d'une enzyme digestive qui est la pepsine, il est prévu une température prédéterminée de 45°C à 55°C, ou, pour détecter des nématodes dans du poisson dans le cas d'une enzyme digestive qui est la pepsine, il est prévu une température prédéterminée de 30°C à 40°C.
